**Europäisches Patentamt**

**European Patent Office**

**Office européen des brevets**

(19)

(11) Publication number: **0 056 254**
**A1**

# (12) EUROPEAN PATENT APPLICATION

(21) Application number: **82100086.6**

(22) Date of filing: **08.01.82**

(51) Int. Cl.³: **G 01 N 33/54**

(30) Priority: **14.01.81 US 224984**

(43) Date of publication of application: **21.07.82**
**Bulletin 82/29**

(84) Designated Contracting States: **AT BE CH DE FR GB IT LI NL SE**

(71) Applicant: **Wood, David Eldon, 575 Massar Avenue, San Jose California 95116 (US)**

(72) Inventor: **Wood, David Eldon, 575 Massar Avenue, San Jose California 95116 (US)**

(74) Representative: **Kuhn, Eduard et al, Patentanwälte Holländer & Kuhn Jungfernstieg 38, D-2000 Hamburg 36 (DE)**

(54) **Treatment of insoluble surfaces to inhibit nonspecific protein binding.**

(57) Compositions for inhibiting nonspecific binding protein to water insoluble surfaces, where specific binding of protein is involved. Specifically, hydrophilic polysaccharides, normally soluble in water, are covalently bonded to the water insoluble surface to substantially completely coat the surface. A biological substance may then be coupled to the polysaccharide and permit specific binding of protein to the covalently bonded biological substance and easy removal of nonspecifically bound protein. The compositions find use in competitive protein binding assays, cytology, serology, agglutination assays and the like.

EP 0 056 254 A1

## Description

Treatment Of Insoluble Surfaces To
Inhibit Nonspecific Protein Binding

### Technical Field

This invention relates to biological or immunological substances attached to solid carriers for use in diagnostic tests, enzyme processes, affinity purifications, and the like.

### Background Art

Soluble biological substances attached to solid carriers have many uses in diagnostic tests, enzyme processes, and affinity purifications. For example, attachment of antibodies or antigens to a solid carrier allows their immunological partners to be easily removed from a mixture of many substances. Similarly, attaching enzymes to a solid carrier allows them to be easily removed from the reaction mixture or to be used in a continuous flow process. Heterogenous radioimmunoassays and enzyme immunoassays rely on attachment of one or more of the reactants to a solid phase to enable separation from the free reactants. Agglutination assays (to determine the presence of an antigen or antibody in a fluid) utilize indicator or carrier particles (upon which are carried the appropriate immunological material) in order to make the immunological complex more easily visible. Separation and identification of cells, cellular constituents, and bacteria are aided by antibodies or antigens coupled to solids. Biological particles will, for example, specifically adhere to solids coated with appropriate antibodies and antigens so that separation from other particles can be affected. Identification of biological particles can be made through the specific adherence of small particles coated with appropriate antibody or antigen. These small particles can incorporate a substance such as a fluorescent dye, radioactive tracer, or electron dense substance which makes their presence more readily detectable.

Two of the major difficulties in the use of solid carriers as described above are reliably attaching the soluble biological substances and preventing nonspecific sticking of undesired substances to the carrier. The consequences of these problems include excessively high background and low sensitivity in assays and loss of material and low purity in affinity purifications and enzyme processes.

The solution to the first of these problems has been approached through covalent bonding of proteins and peptides to polymer solids. For example, U.S. Patent No. 3,645,852 discloses a process wherein cyanogen halides are used to activate a water insoluble polymer which then couples to a water soluble protein. Water soluble carbodiimides can be used as a condensing agent to bind protein to polymeric carrier particles according to U.S. Patent No. 3,857,931. Biological substances can be covalently bound to plastic materials whose surfaces have been coated with glutaraldehyde. (See U.S. Patent No. 4,001,583). In U.S. Patent No. 4,046,723 a three step method is revealed for coupling proteins to a latex having surface carboxylic amide groups. A process for the manufacture of protein or peptide polystyrene latex compounds is described in U.S. Patent No. 4,118,349 in which the linkage  is effected by means of an aromatic diazonium compound. A two step process is disclosed in U.S. Patent No. 4,140,662 which links immunological substances to latex polymers via reactions with a diamine mediated by a carbodiimide followed by reaction with a bifunctional aldehyde. These and other methods known to couple biological substances to polymer materials (see for example, Kiefer "The Chemical Modification of Proteins, Haptens, and Solid Supports", Immunological Methods, Academic Press, 1979, pp.137150) are undoubtedly more generally reliable than the hydrophobic bonding which was used prior to the covalent bonding methods. However, they do not alleviate the problem of nonspecific sticking and sometimes make it worse.

United Kingdom patent application No. 33894/77 discloses an invention which solves some of the above problems by covalently bonding a water-soluble polyhydroxy compound, preferably an amino polysaccharide, to a latex carrier, preferably a carboxylated polymer, via a water-soluble carbodiimide, the Woodward reagent K(N-ethyl-5-phenyl-isoxazolium-3'-sulfonate) or a water-soluble chloroformiate. The amino groups of the amino polysaccharide which are not bonded to the latex carrier are converted to hydroxyl groups. Then the polysaccharide is activated with periodate to oxidize some of the glucose rings to dialdehydes. Thereafter, an immunological active material is reacted with the thus activated polysaccharide. The reaction with the immunologically active material must be performed shortly after formation of the dialdehydes because the dialdehyde containing polysaccharide is subject to relatively fast degradation. Thus, preactivated latex-polysaccharide particles cannot be readily stored or shipped to an ultimate user who would then be able to attach any desired immunologically active material.

## Disclosure Of Invention

Water insoluble surfaces, particularly particles or vessel surfaces, are coated with polysaccharide coatings, by covalent bonding, generally via a cyanuric halide moiety, to form a polysaccharide coated surface. The polysaccharides are normally high molecular weight and water soluble. The polysaccharide coating may then be used for covalently bonding via a cyanuric halide moiety to a biological substance which is in turn to be used for binding to a protein in a heterogenous protein composition. Easy separation may then be obtained between specifically and nonspecifically bound protein. Also, materials having polysaccharide coated surfaces may be reacted with a cyanuric halide to form an activated reactant which is storable and shippable, whereby the biological substance may be attached by the ultimate user.

## Best Mode For Carrying Out The Invention

Novel solid materials are provided, as particles, surface walls, or the like which can be used in the separation of protenaceous mixtures.

- 5 -

Particles will generally be 0.2μ to 1cm in diameter. Specifically, a water insoluble surface is coated with a water soluble polysaccharide by covalently bonding the water soluble polysaccharide to the surface using, most preferably, a cyanuric halide moiety or linking group. A biological substance may then be bonded to the polysaccharide by, most preferably, a cyanuric halide moiety or linking group. In any event, at least one of such bondings must be via a cyanuric halide moiety. The resulting product may then be used for isolation of a specific protein or group of proteins from a heterogenous mixture of proteins. Normally, the compositions will be used where specific binding properties between a ligand and a receptor are involved and one of the members of the specific binding pair is to be segregated from other proteins. When the term "cyanuric halide" is used herein it includes cyanuric chlorides, bromides, and iodides. The term "cyanuric halide moiety" means the group remaining after one or more of the halides have been replaced by a link to another compound, e.g., to the water insoluble surface, to the polysaccharide, or to a biological substance.

A large number of particles have been used for a variety of purposes and linked to a variety of groups. Of particular interest are latex particles as described in U.S. Patent Nos. 4,046,723, 4,118,349 and 4,140,662. Glass surfaces are described in U.S. Patent No. 4,169,138. Other polymers may be found in U.S. Patent Nos. 3,619,371, 3,700,609 and 4,201,763. In each of these cases, a wide variety of linking groups are disclosed for bonding to various biological substances, particularly proteins.

In the embodiment of the invention wherein the polysaccharide coated surface is activated with a cyanuric halide moiety, the manner in which the water insoluble surface is conjugated to the polysaccharide will depend upon the nature of the functional groups of the polysaccharide. Where only hydroxylic groups are available, for the most part ethers or esters will be formed, employing active halogen or nonoxocarbonyl groups. Alternatively, and highly preferred, is employing polysaccharides which have a plurality of amino functionalities. The amino functionalities may be naturally present as part of a glucosamine, for example, or be introduced by modification of the polysaccharide with a group having a free amino group. Modification of polysaccharides with free amino groups is well known in the literature and an ample number of such polysaccharides are commercially available. The activating cyanuric halide moiety may be linked to the polysaccharide coated surface via the amino groups, hydroxyl groups or other active hydrogen containing groups.

The polysaccharides which are employed are characterized by being water soluble, relatively high molecular weight, normally in excess of 5,000 daltons, more usually in excess of 10,000 daltons, and may be 1,000,000 daltons or higher. The polysaccharide may be a polymer or copolymer of glycose(s), e.g., glucose and fructose, a mixture of carbohydrates, such as neuraminic acids, uronic acids, glycosamines, or the like.

In addition, the polysaccharide may be a combination of block or alternating copolymers or combinations thereof of saccharides and condensation monomers, particularly epoxides. Polysaccharides of particular interest include dextran, Ficoll, (a synthetic copolymer of sucrose and epichlorohydron, Pharmacia Fine Chemicals, Piscataway, New Jersey), agarose, hyaluronic acid, etc.

Of particular interest is the presence of an amino group, normally being bonded to a short alkylene chain of from about 2 to 6 carbon atoms, which are bonded to functionalities of the polysaccharide. Particularly convenient is the reaction of diamines with carboxyl functionalities present on the polysaccharide. See Inman, J. of Immunology 114, 704-709 (1975).

In the embodiment of the invention wherein a cyanuric halide moiety is used to activate the polysaccharide coated surface, the polysaccharides may be coupled to carboxyl functionalized polymers by using water soluble carbodiimides, or other compounds as linking agents. Very preferably, however, a cyanuric halide is used as the linking agent. The biological substances are then coupled via their carboxyl groups to the polysaccharide layer through remaining amine groups by use of a cyanuric halide moiety, most preferably cyanuric chloride. In the most preferred embodiment of the invention a cyanuric halide moiety is used to couple the amine functionalized polysaccharide to amine functionalized polymers and also to amine containing biological substances.

A wide variety of proteins and polypeptides find use in linking to the polysaccharides.

Illustrative of proteins are enzymes, antibodies, natural receptors e.g., thyroxine binding globulin and avidin, globins e.g., hemoglobin, ocular lens proteins, surface antigens, histo-compatibility antigens, and the like.

The subject compositions can be used wherever an insoluble material is used for specific binding to a protein present in a mixture. This situation is encountered in competitive protein binding assays, cell sorting, cytology, histology, and the like. Since the procedures will vary widely, the subject invention generally involves combining the insoluble material, as a particle or surface of a larger structure, with the protein mixture and allowing a sufficient time for binding between the biological substance on the surface and the specific protein binding partner. The solid surface is then washed free of nonspecifically bound protein, leaving only specifically bound protein.

Of particular interest are situations employing particles, which may be labeled or unlabeled. The labels may include radioactive isotopes, fluorescers, magnetic materials, enzymes, enzyme substrates, or the like. The labels may be bonded to the water insoluble surface, the polysaccharide, or the biological substance, desirably being bonded to the water insoluble surface or the polysaccharide.

The following examples are by way of illustration and not by way of limitation.

## EXPERIMENTAL

Polystyrene Microspheres

Nitration: Suspend 5 grams of 1 micron diameter polystyrene microspheres (Polysciences Inc., Warrington, PA) in 100 ml ice cold 1:1 $HNO_3:H_2SO_4$. Stir the suspension for 30 minutes in an ice bath, then quench by pouring into 1 L ice cold water. Wash the nitrated microspheres in water three times by centrifugation.

Reduction: Suspend the nitrated microspheres in 110 g $SnCl_2 \cdot 2H_2O$ dissolved in 100 ml concentrated HCl and stir at room temperature for 10 hours. Separate the reduced microsphere from the $SnCl_2$ solution and wash two times in water by centrifugation. Wash the microspheres in turn in $H_2O$, 0.1N HCl, $H_2O$, 0.1N NaOH, and $H_2O$.

Activating the reduced microspheres with Cyanuric Chloride: Suspend the reduced microspheres in 900 ml cold $H_2O$ and disperse by sonication in a stainless steel ultrasonic bath cleaner. Add 0.5g cyanuric chloride dissolved in 150 ml water: alcohol 2:1 and incubate with intermittent sonication for 30 minutes. Centrifuge the suspension to remove the microspheres and wash twice with cold water by centrifugation.

Coating the microspheres with N-(2-aminoethyl Carboxymethylated Ficoll (AECM-Ficoll): Suspend the cyanuric chloride coupled microspheres in 1 L of cold water by sonication and add 1.2g AECM-Ficoll dissolved in 50 ml $H_2O$. Incubate with intermittent sonication for 12 hours. Wash the AECM-Ficoll coupled microspheres twice in water by centrifugation. Vacuum filter through Whatman #1 paper to remove any clumped microspheres.

Activating the coated microspheres with Cyanuric Chloride: Suspend the AECM-Ficoll coupled microspheres in 750 ml water, cool to 0°C and add 1g cyanuric chloride dissolved in 200 ml cold 1:1 alcohol:water. Incubate at 0°C for 35 minutes and then separate the activated microspheres from the suspension by centrifugation. Wash in cold water three times by centrifugation. These activated microspheres will now couple to proteins incubated with them at room temperature. They can also be stored, generally at about 0°C, for months without significant deterioration.

Polystyrene Macrospheres

Nitration: Cover about 250 3/8" diameter polystyrene spheres with 120 ml 0°C 2:1 $H_2SO_4$:$HNO_3$ and incubate with gentle mixing at 0°C for 15 minutes. Pour off the mixed acid and rinse spheres with 0°C 50% $H_2SO_4$, then with ice cold water until wash water tests neutral.

Reduction: Cover the nitrated spheres with 110 g $SnCl_2 \cdot 2H_2O$ dissolved in 100 ml conc. HCl and incubate with occasional stirring for 15 hours at room temperature. Decant the $SnCl_2$ solution and wash the spheres in turn with $H_2O$; 0.1$\underline{N}$ HCl, $H_2O$, 0.1$\underline{N}$ NaOH and $H_2O$.

Activating with Cyanuric Chloride: Cover the reduced spheres with 0.1g cyanuric chloride dissolved in 10 ml alcohol and 200 ml $H_2O$ and incubate at room temperature for 40 minutes. Decant the cyanuric chloride solution and wash the spheres with several portions of water.

Coating with AECM-Ficoll:  Cover the
cyanuric chloride coupled spheres with 0.14g AECM-
Ficoll dissolved in 120 ml $H_2O$ and incubate with
occassional stirring for 12 hours.  Decant the AECM-
Ficoll solution and wash the spheres two times with
cold water.

Activating the coated spheres with
Cyanuric Chloride:  Cool the Ficoll coupled
spheres to 0°C and cover with 150 ml ice cold
water containing 0.18g cyanuric chloride and 18
ml alcohol.  Incubate at 0°C for 30 minutes.
Decant the cyanuric chloride solution and wash
the spheres three times in ice cold water.  The
spheres will now react to form permanent covalent
linkage with protein macromolecules such as IgG,
enzymes, etc.  They can be stored for several
months at about 0°C without significant
deterioration.

Testing Microspheres for Protein
Binding:  To 0.127mg of the cyanuric chloride
activated AECM-Ficoll microspheres prepared above
was added sufficient MOPC-21 myeloma protein to
make the final protein concentration 3  g/ml in a
total volume of 1:1 ml normal saline.  After one
hour incubation at room temperature the unbound
protein was removed by three wahses in normal
saline via centrifugation.  The MOPC-21 coupled
microspheres were then incubated for one hour at
room temperature in 1 ml saline containing 0.642
mg/ml $I^{125}$ labelled monoclonal antibodies, anti-
4a.  After washing the unbound labelled protein
from the microspheres with saline containing 0.1%
BSA by centrifugation three times, the bound label was counted. The result was 0.013% anti-4a by weight which corresponds to about 200 IgG molecules per microsphere.

Industrial Applicability

In accordance with one embodiment of the subject invention, novel compositions are provided comprising water insoluble surfaces coated with covalently bonded (via cyanuric halide moieties) water soluble polysaccharides, normally amino functionalized, which are linked to the water insoluble surface. In accordance with another embodiment of the invention polysaccharide coated water insoluble surfaces are bonded to a cyanuric halide moiety to form an activated composition which is storable and which can be readily attached to a biological substance via the cyanuric halide moiety. The two embodiments are preferably used in combination. By covalently bonding biological substances, particularly poly(amino acids), separations can be achieved in proteinaceous mixtures by specifically binding the binding partner of the biologial substance to the biological substance bound to the insoluble support. Non-specifically bound proteins may be readily separated to provide a clean accurate separation between the specifically bound protein and nonspecific protein.

Although the foregoing invention has been described in some detail by way of illustration and example for purposes of clarity of understanding, it will be obvious that certain changes and modifications may be practiced within the scope of the appended claims.

## Claims

1. Composition useful for specifically binding to a specific binding protein, which is a specific binding partner to a biological substance, where said protein is associated with other proteins, said composition comprising:
a water insoluble support; and
a polysaccharide coating covalently bonded through a cyanuric halide moiety to such support and substantially completely covering the surface of said support to be exposed to said specific binding protein.

2. The composition as set forth in claim 1, including the biological substance which is the binding partner to said specific binding protein covalently bonded via a second cyanuric halide moiety to said polysaccharide.

3. A composition as set forth in claim 2, wherein said water insoluble support is a plastic.

4. A composition as set forth in claim 2, wherein said water insoluble support is glass.

5. A composition as set forth in claim 2, wherein said support is a particle and said composition is labelled with a label capable of providing a detectable signal.

6. Activated composition which is storage stable and useful for specifically binding to a specific binding protein, which is a specific binding partner to a biological substance, where said protein is associated with other proteins, said composition comprising:

an amino functionalized water insoluble support; and

a polysaccharide coating covalently bonded to said support and substantially completely covering the surface of said support to be exposed to said specific binding protein; and

a cyanuric halide moiety covalently bonded to said polysaccharide coating.

7. A composition as set forth in claim 6, further including:

a biological substance which is the binding partner to said specific binding protein covalently bonded to said cyanuric halide moiety.

8. A composition as set forth in claim 6, wherein said support is polystyrene.

9. In a method for separating a specifically binding protein from nonspecifically binding protein, the improvement which comprises:

employing a composition according to any of claims 1 or 6, wherein said biological substance is the specific binding partner of said protein.

0056254

10. A method of preparing a water insoluble surface of a solid support specifically binding to a specific binding protein, which is a specific binding partner to a biological substance, where said protein is associated with other proteins, comprising:

reacting a cyanuric halide with said water insoluble surface of said solid support, to attach a cyanuric halide moiety thereto; and

covalently bonding sufficient of a polysaccharide with said cyanuric halide moiety to substantially completely cover said surface of said support to be exposed to said specific binding protein.

11. A method as set forth in claim 10, wherein said polysaccharide comprises an amino functionalized polysaccharide.

12. A method as set forth in claim 10, further including:

reacting said polysaccharide covered surface of said support with an additional cyanuric halide to provide an activated storage stable composition having an additional cyanuric halide moiety covalently bonded thereto.

13. A method of converting a water insoluble surface of a solid support into an activated storage stable composition useful for specifically binding to a specific binding protein, which is a specific binding partner to a biological substance, where said protein is associated with other proteins, comprising:

covalently bonding a sufficient amount of a polysaccharide to said surface of said support to substantially completely cover said surface of said support to be exposed to said specific binding protein; and

reacting said polysaccharide covered surface of said support with a cyanuric halide to provide said activated storage stable composition with a cyanuric halide moiety covalently bonded thereto.

14. A method as set forth in claim 13, wherein said polysaccharide comprises an amino functionalized polysaccharide.

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X Y | GB-A-2 004 892 (F.HOFFMANN-LA ROCHE & CO.) *Page 1, line 56 - page 3, line 109; page 4, line 43 - page 5, line 38; examples; claims 1,6,11,14,18,27,33* | 1-14 | G 01 N 33/54 |
| | --- | | |
| D,Y | US-A-3 645 852 (R.E.A.V.AXEN et al.) *The entire document* | 1-14 | |
| | --- | | |
| Y | US-A-4 217 338 (G.A.QUASH) *Column 1, line 1 - column 2, line 27; column 2, line 33 - column 4, line 68; column 6, examples 1-8 and 30-38; claims 1-3,10,12-17* | 1-9 | |
| | --- | | **TECHNICAL FIELDS SEARCHED (Int. Cl. 3)** |
| A | CHEMICAL ABSTRACTS, vol. 79, no. 9, 3rd September 1973, page 230, column 2, no. 51610n, Columbus Ohio (USA); J.D.SIPE et al.: "Preparation of solid-phase immunosorbents by coupling human serum proteins to cyanogen bromide-activated agarose". & APPL. MICROBIOL., vol. 25, no. 6, 1973, pages 880-884. *Abstract* | | G 01 N |
| | --- | | |
| A | GB-A-2 027 031 (F.HOFFMANN-LA ROCHE & CO.) | | |
| | --- | | |

The present search report has been drawn up for all claims

| Place of search THE HAGUE | Date of completion of the search 20-04-1982 | Examiner GRIFFITH G. |
|---|---|---|

European Patent
Office

**EUROPEAN SEARCH REPORT**

0056254
Application number

EP 82 10 0086

| | DOCUMENTS CONSIDERED TO BE RELEVANT | | Page 2 |
| --- | --- | --- | --- |

| Category | Citation of document with indication, where appropriate, of relevant passages | Relevant to claim | CLASSIFICATION OF THE APPLICATION (Int. Cl. 3) |
| --- | --- | --- | --- |
| A | GB-A-2 005 275 (F.HOFFMANN-LA ROCHE & CO.) | | |
| | --- | | |
| A | GB-A-1 248 765 (PHARMACIA AB) | | |
| | ----- | | |
| | | | TECHNICAL FIELDS SEARCHED (Int. Cl. 3) |
| | The present search report has been drawn up for all claims | | |

| Place of search THE HAGUE | Date of completion of the search 20-04-1982 | Examiner GRIFFITH G. |
| --- | --- | --- |